# EUROPEAN PATENT SPECIFICATION

(11) **EP 3 389 812 B1**
(45) Date of publication and mention of the grant of the patent: **05.11.2025**
(21) Application number: 16819386.0
(22) Date of filing: 30.11.2016
(51) Int. Cl.: B01D 3/14, B01D 3/36, B01D 11/00, B01D 3/42, C07C 255/08, C07C 253/34

(54) **RECOVERY COLUMN FOR THE PURIFICATION OF ACRYLONITRILE/ACETONITRILE MIXTURES**
RÜCKGEWINNUNGSSÄULE ZUR REINIGUNG VON ACRYLNITRIL-/ACETONITRIL-GEMISCHEN
COLONNE DE RÉCUPÉRATION POUR LA PURIFICATION DE MÉLANGES D'ACRYLONITRILE/D'ACÉTONITRILE

(30) Priority: 17.12.2015 CN 201510947392
(43) Date of publication of application: 24.10.2018
(73) Proprietor: Ineos Europe AG, 1180 Rolle, Vaud (CH)
(72) Inventor: MCDONEL, Timothy Robert, Elburn Illinois 60119 (US); COUCH, Jay Robert, Naperville Illinois 60564 (US); WACHTENDORF, Paul Trigg, Victoria Texas 77904 (US)
(74) Representative: King, Alex
(86) International application number: PCT/US2016/064069
(87) International publication number: WO 2017/105831

(56) References cited:
- EP-A2- 0 115 262
- WO-A1-2015/183866
- DE-A1- 1 920 083
- FR-A- 1 427 860
- US-A- 3 264 197
- US-A- 3 328 268
- US-B2- 8 212 067

## Description

A process is provided for separating acrylonitrile from a mixture of acrylonitrile and acetonitrile. More specifically, the process includes contacting the mixture with aqueous solvent to provide an acrylonitrile-water azeotrope and separating the acrylonitrile-water azeotrope from the acetonitrile.

### BACKGROUND

Recovery and purification systems for acrylonitrile are known, see for example U.S. Pat. Nos. 4,234,510; 3,936,360; 3,885,928; 3,433,822; and 3,399,120. Typically, propylene, ammonia and air are reacted in a vapor phase with an ammoxidation catalyst. The vaporous reactor effluent is then passed to a quench system wherein the reactor effluent is directly contacted with an aqueous quenching liquid, usually water. This quenching removes unreacted ammonia and heavy polymers. The quenched gases then proceed to an absorption column.

In the absorber, the gases are directly contacted with an absorbing liquid, again usually water. The water, acrylonitrile, acetonitrile, HCN and associated impurities leave the bottom of the absorber in an aqueous solution. Inert gases are removed from the top of the absorber. The aqueous solution then proceeds to a recovery column. This column removes acetonitrile from the aqueous solution through extractive distillation. Examples of systems and processes which involve a recovery column for separating acetonitrile from acrylonitrile, and in particular producing overhead, bottom and side streams from the recovery column, include those disclosed in WO 2015/183866, DE1920083 and US 8212067.

### SUMMARY

The present invention provides a process for separating acrylonitrile from a mixture of acrylonitrile and acetonitrile, the process comprising: providing a mixture of acrylonitrile and acetonitrile to a recovery column and contacting the mixture of acrylonitrile and acetonitrile with aqueous solvent to provide an acrylonitrile-water azeotrope; maintaining a temperature of 55 to 80 °C in a top portion of a top section of the recovery column; maintaining a temperature of 65 to 85 °C in a top portion of a middle section of the recovery column and a temperature of 100 to 120 °C in a bottom portion of the middle section of the recovery column; maintaining a temperature of 105 to 125 °C in a bottom portion of a bottom section of the recovery column; and separating the acrylonitrile-water azeotrope from the acetonitrile to provide an overhead stream that includes the acrylonitrile-water azeotrope, a bottoms stream, and a sidestream, and wherein the recovery column includes a crude acetonitrile concentration zone which includes an internal vertical baffle and an upper outlet configured to allow the sidestream to flow out of the recovery column from the crude acetonitrile concentration zone, and further wherein the overhead stream includes the acrylonitrile-water azeotrope, 0.05 weight percent or less acetonitrile and has 70 to 90 weight percent acrylonitrile: the bottoms stream includes 0 to 0.0075 weight percent acetonitrile; and the sidestream includes 5 to 70 weight % acetonitrile.

### BRIEF DESCRIPTION OF FIGURES

The features and advantages of the process will be more apparent from the following figures.
Figure 1 provides a general view of a recovery column;
Figure 2 illustrates another view of a recovery column;
Figure 3 illustrates a recovery column that includes an acetonitrile concentration zone; and
Figure 4 illustrates a recovery column temperature profile.

Corresponding reference characters indicate corresponding components throughout the several views of the drawings. Skilled artisans will appreciate that elements in the figures are illustrated for simplicity and clarity and have not necessarily been drawn to scale. For example, the dimensions of some of the elements in the figures may be exaggerated relative to other elements to help to improve understanding of various aspects. Also, common but well-understood elements that are useful or necessary in a commercially feasible aspect are often not depicted in order to facilitate a less obstructed view of these various aspects.

### DETAILED DESCRIPTION

The following description is not to be taken in a limiting sense, but is made merely for the purpose of describing the general principles of exemplary embodiments. The scope of the invention should be determined with reference to the claims.

### Recovery Column

Any type of recovery column may be utilized in connection with the present process. Several types of recovery column configurations are described herein as examples.

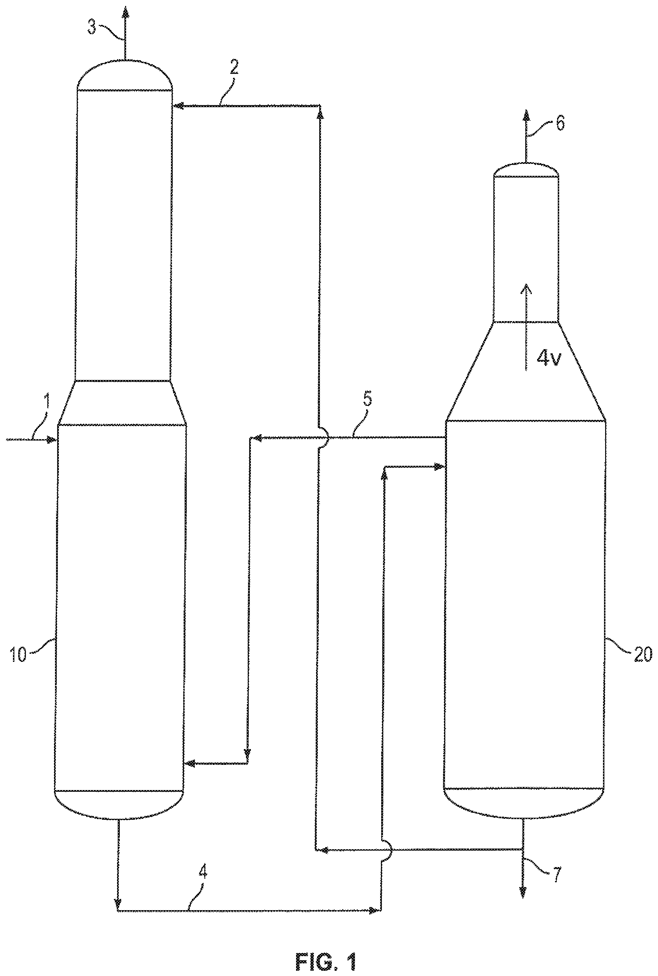

A conventional process for separation of acrylonitrile from acetonitrile is shown in FIG. 1. As shown in FIG. 1, feed stream 1 from an acrylonitrile absorber (not shown) is sent to first column 10. Feed stream 1 typically contains acrylonitrile, hydrogen cyanide (HCN), acetonitrile, and water. Water stream 2 that is substantially free of acetonitrile is recycled from at or near the bottom of second column 20 to an upper portion of first column 10 in order to facilitate the separation of acetonitrile from acrylonitrile and HCN by extractive distillation. Stream 3 containing acrylonitrile, HCN, and a portion of the water from feed 1 is removed from the top of first column 10. Liquid stream 4 containing water and acetonitrile is sent as feed from the bottom of first column 10 to second column 20. Vapor stream 5 from second column 20 is sent to first column 10 to provide the heat needed for distillation in first column 10. Vapor sidestream 4v moves up the second column 20 and includes acetonitrile. A crude acetonitrile stream 6 containing acetonitrile, water and small amounts of acrylonitrile and HCN is removed from the top of second column 20. The remaining water stream 7, which is substantially free of acrylonitrile, HCN, and acetonitrile and is not recycled as water stream 2 back to first column 10, is discharged from second column 20 at or near the bottom of second column 20.

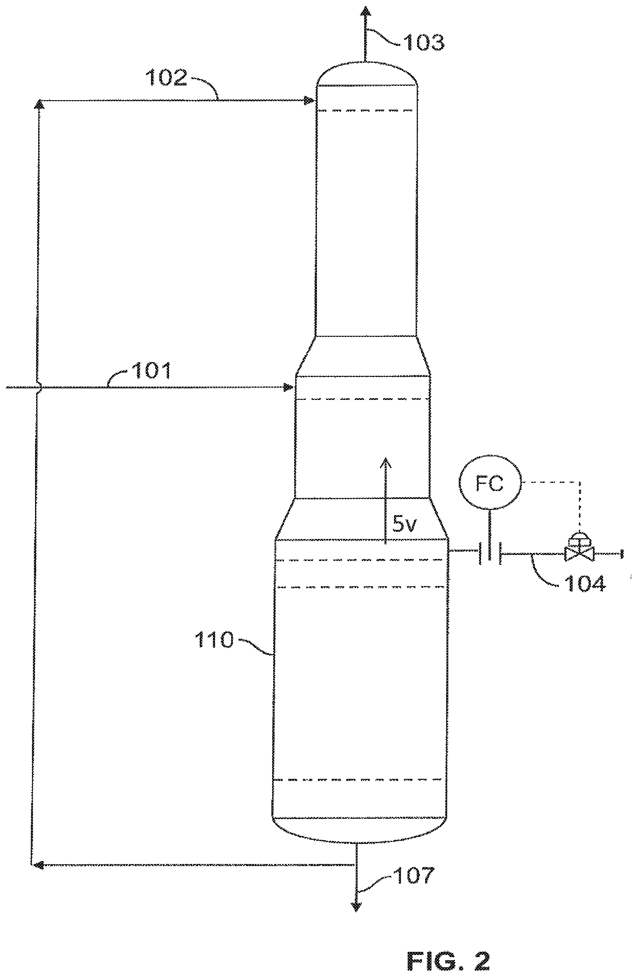

Another conventional process for separation of acrylonitrile from crude acetonitrile is shown in FIG. 2. As shown in FIG. 2, feed stream 101 from an acrylonitrile absorber (not shown) is sent to column 110. Feed stream 101 typically contains acrylonitrile, hydrogen cyanide (HCN), acetonitrile, and water. Bottoms stream 102 that is substantially free of acetonitrile is recycled from at or near the bottom of column 110 to the top of column 110 in order to facilitate the separation of acetonitrile from acrylonitrile and HCN by extractive distillation. The portion of bottoms stream 102 that is not recycled to the top of column 110 is discharged from column 110 as stream 107. Overhead stream 103 containing acrylonitrile, HCN, and a portion of the water from feed stream 101 is removed from the top of column 110. A vapor sidestream 5v moves up column 110 and a sidestream 104 (corresponding to 4v in FIG. 1) containing water and acetonitrile is removed from column 110.

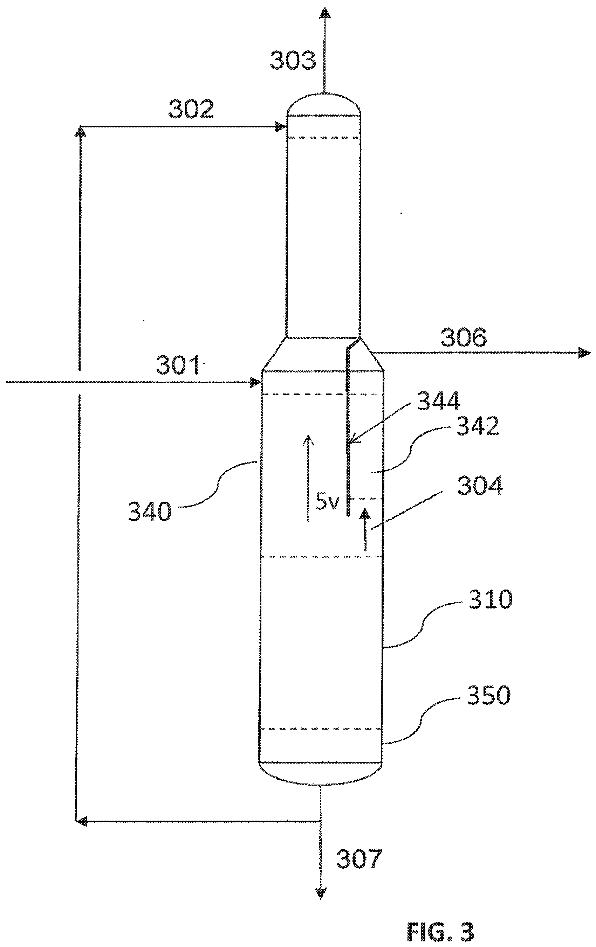

An example of a recovery column with an acetonitrile concentration zone (342) is shown in FIG. 3. In this aspect, apparatus 300 includes column 310. Column 310 includes a top section 330, a middle section 340, and a bottom section 350. Middle section 340 of the single column 310 may be configured to receive feed stream 301. In one aspect, the ratio of a diameter of the middle section to the top section is about 0.8 to about 1.2, in another aspect about 0.9 to about 1.1, in another aspect about 1.5 to about 2.5, in another aspect, about 1.75 to about 2.25, and in another aspect, about 1.8 to about 2. In one aspect, the ratio of a diameter of the bottom section to the middle section is about 0.8 to about 1.2, in another aspect about 0.9 to about 1.1, in another aspect about 1.5 to about 2.5, in another aspect, about 1.75 to about 2.25, and in another aspect, about 1.8 to about 2. In one aspect, the ratio of a diameter of the bottom section to the top section is about 0.8 to about 1.2, in another aspect about 0.9 to about 1.1, in another aspect about 1.5 to about 2.5, in another aspect, about 1.75 to about 2.25, and in another aspect, about 1.8 to about 2. In one aspect, the top section, middle section and bottom section are each about 25 to about 40% of a height (tangent to tangent) of the recovery column.

As shown in FIG. 3, overhead stream 303 containing acrylonitrile, HCN and water is removed from the top of column 310. The recovery column includes a crude acetonitrile concentration zone 342. The crude acetonitrile concentration zone 342 includes internal vertical baffle 344. The crude acetonitrile concentration zone may include a plurality of trays. The trays are located at different heights of the column, and each tray includes a horizontal plane extending across a cross section of crude acetonitrile concentration zone 342. Crude acetonitrile concentration zone 342 includes an upper outlet configured to allow sidestream 306 to flow out of column 310 of crude acetonitrile concentration zone 342. Vapor may flow up either side of baffle 344 as stream 304 or as vapor stream 5v. A portion of bottoms stream 302 that is not recycled to the top of column 310 is discharged as stream 307.

The recovery column includes a top section, middle section and bottom section. In one aspect, the top, middle and bottom sections are each about 25 to about 40% of a height (tangent to tangent) of the recovery column. Each recovery column section may be further divided into portions. For example, the middle section of the recovery column includes a top portion and a bottom portion, and may include a middle portion. In this aspect, the top portion, middle portion and bottom portion are each about 25 to about 40 % of a height of the middle section of the recovery column. In another aspect, the top section of the recovery column includes a top portion and a bottom portion that are each about 40 to about 60% of a height of the top section of the recovery column. In another aspect, the bottom section of the recovery column includes a top portion and a bottom portion that are each about 40 to about 60% of a height of the bottom section of the recovery column.

In another aspect, the recovery column may include about 80 to about 120 trays, and in another aspect, about 80 to about 100 trays. The plurality of trays in the recovery column includes a top section of trays, a middle section of trays, and a bottom section of trays. In this aspect, the top section of trays, middle section of trays and bottom section or trays are each about 25 to about 40% of a total number of trays in the recovery column.

The top section of trays of the recovery column includes a top portion of trays and a bottom portion of trays that are each about 40 to about 60% of a total number of trays in the top section of trays in the recovery column. The middle section of trays includes a top portion of trays, middle portion of trays and bottom portion of trays. The top portion of trays, middle portion of trays and bottom portion of trays are each about 25 to about 40% of a total number of trays of the middle section of trays of the recovery column. The bottom section of trays of the recovery column includes a top portion of trays and a bottom portion of trays that are each about 40 to about 60% of a total number of trays in the bottom section of trays in the recovery column.

### Recovery Column Operation

A feed stream provided to the recovery column typically contains acrylonitrile, hydrogen cyanide (HCN), acetonitrile, and water (shown as feed stream 1 in FIG. 1, as feed stream 101 in FIG. 2, and as feed stream 301 in FIG. 3). In this aspect, the feed stream provided to the recovery column includes about 2 to about 10 weight % acrylonitrile, in another aspect, about 3 to about 7 weight %, and in another aspect, about 4 to about 6 weight % acrylonitrile. The mixture also includes acetonitrile in an amount of about 0.1 to about 0.3 weight % acetonitrile, and in another aspect, about 0.15 to about 0.25 weight % acetonitrile. The mixture may also include other components, such as for example, acrolein and/or oxazole in smaller amounts.

Solvent water enters the recovery column at a top portion of the column (shown as water stream 2 in FIG. 1, water stream 102 in FIG. 2 and water stream 302 in FIG. 3). Solvent water which is introduced to the top of the recovery column flows down the tower across the trays, condensing and extracting acetonitrile as it travels to the bottom of the recovery column. As the liquid descends across the trays in the recovery column to the tower bottom, hot vapor from a stripper and heat from the live steam added, travel up the tower through the tray holes allowing intimate contact with the liquid on them. Heat transfer takes place between the liquid and vapor traffic and tends to move all the organics (except acetonitrile) up to the column top.

The overhead stream produced by the recovery column (shown as stream 3 in FIG. 1, stream 103 in FIG. 2, and stream 303 in FIG. 3) may include acrylonitrile, HCN, and a portion of the water from the feed stream. The recovery column provides a vapor sidestream that includes water and acetonitrile that is substantially free of acrylonitrile and HCN (shown as stream 104 in FIG. 2, and 306 in FIG. 3). The recovery column may also provide a bottoms stream (shown as bottoms stream 2 in FIG. 1, bottoms stream 102 in FIG. 2, and bottoms stream 307 in FIG. 3) which exits from a bottom portion of the recovery column. At least a portion of the bottoms stream may be recycled back to a top portion of the recovery column. Known methods for heating the recovery column include for example, steam, steam heated reboiler and/or heat exchange with other process streams.

### Temperature Control

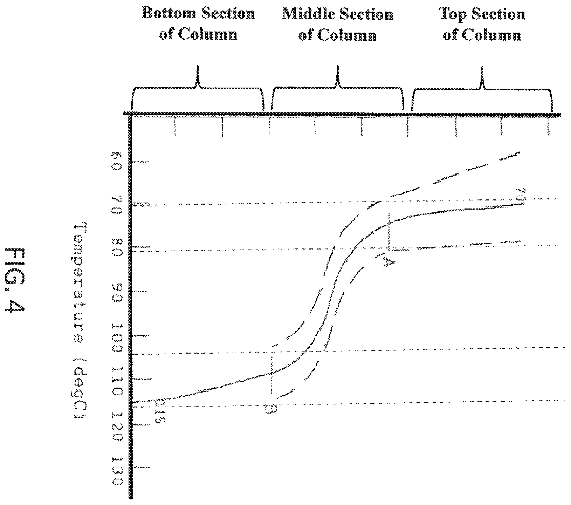

Figure 4 describes a temperature profile for recovery column operation. Proper temperature control and a temperature profile as described in Figure 4 are important for proper column operation. Variations within the temperature profile as defined by the dotted lines in Figure 4 can be effected by a combination of changes involving solvent water and feed temperatures, solvent water rate, and steam rate. Column pressure can also change the profile.

Changes above point "A" on the curve are predominantly due to variations in composition and pressure. Temperature variations above point "A" are not critical, but the overhead temperature should be kept as low as possible to minimize amounts of oxazole, acetonitrile, acetone and water going overhead. The temperature variations below point "B" are predominantly due to tray pressure changes, since the composition in this section is mostly water.

Temperature control of the recovery column includes known temperature control systems that can include reboilers and heat exchangers. In one aspect, the heat duty required to produce the necessary boil-up in the bottom of the recovery column may be provided by heat transfer in any conventional reboiling apparatus. Conventional reboilers may include some variant of a shell-and-tube exchanger. Some examples of reboiler configurations include kettle, thermosyphon, forced circulation, stab-in bundle, horizontal, vertical and falling film. In one aspect, the process includes controlling temperature by removing liquid at or near the bottom of the recovery column and exchanging the liquid in a thermosiphon reboiler. In this aspect, the effluent from the thermosiphon reboiler is returned to the recovery column. Live steam may be injected either to supplement or to replace the required heat duty of the recovery column. In another aspect, the process includes reboiling of the recovery column through two vertical thermosyphon reboilers in parallel, using pressurized steam derived from turbine exhaust.

In one aspect, recovery column temperature is maintained as follows:
the top portion of the middle section of the recovery column is maintained at a temperature of about 65 to about 85 °C, and in another aspect, about 70 to about 80 °C;
the bottom portion of the middle section of the recovery column is maintained at about 100 to about 120 °C, and in another aspect, about 105 to about 115 °C;
the top portion of the top section of the recovery column is maintained at about 55 to about 80 °C, and in another aspect, about 60 to about 75 °C;
the top portion and bottom portion of the top section of the recovery column have a temperature difference of about 0 to about 20 °C, and in another aspect, about 5 to about 15 °C;
the bottom portion of the bottom section of the recovery column is maintained at about 105 to about 125 °C, and in another aspect, about 110 to about 120 °C; and
the top portion and bottom portion of the bottom section of the recovery column have a temperature difference of about 0 to about 15 °C, and in another aspect, about 7 to about 13 °C.

In another aspect, recovery column temperature is maintained as follows:
the top portion of trays of the middle section of the recovery column is maintained at a temperature of about 65 to about 85 °C, and in another aspect, about 70 to about 80 °C;
the bottom portion of trays of the middle section of the recovery column is maintained at about 100 to about 120 °C, and in another aspect, about 105 to about 115 °C;
the top portion of trays of the top section of the recovery column is maintained at about 55 to about 80 °C, and in another aspect, about 60 to about 75 °C;
the top portion of trays and bottom portion of trays of the top section of the recovery column have a temperature difference of about 0 to about 20 °C, and in another aspect, about 5 to about 15 °C;
the bottom portion of trays of the bottom section of the recovery column is maintained at about 105 to about 125 °C, and in another aspect, about 110 to about 120 °C; and
the top portion of trays and bottom portion of trays of the bottom section of the recovery column have a temperature difference of about 0 to about 15 °C, and in another aspect, about 7 to about 13 °C.

The recovery column provides an overhead stream, bottoms stream and sidestream with the following compositions:
an overhead stream that includes the acrylonitrile-water azeotrope and about 0.05 weight percent or less acetonitrile, in another aspect, about 0.03 weight percent or less acetonitrile, and in another aspect, about 0.01 weight percent or less acetonitrile;
an overhead stream that includes about 70 weight percent to about 90 weight percent acrylonitrile, and in another aspect, about 75 to about 85 weight percent acrylonitrile;
a bottoms stream that includes about 0 to about 0.0075 weight percent acetonitrile, in another aspect, about 0.0025 to about 0.007 weight percent acetonitrile, and in another aspect, about 0.0025 to about 0.005 weight percent acetonitrile; and
a sidestream that includes about 5 to about 70 weight percent acetonitrile, in another aspect, about 5 to about 50 weight percent acetonitrile, and in another aspect, about 6 to about 12 weight percent acetonitrile.

## Claims

1. A process for separating acrylonitrile from a mixture that includes acrylonitrile and acetonitrile, the process comprising:
providing the mixture of acrylonitrile and acetonitrile to at least one recovery column (310) and contacting the mixture of acrylonitrile and acetonitrile with aqueous solvent to provide an acrylonitrile-water azeotrope;
maintaining a temperature of 55 to 80 °C in a top portion of a top section (330) of the recovery column (310);
maintaining a temperature of 65 to 85 °C in a top portion of a middle section (340) of the recovery column (310) and a temperature of 100 to 120 °C in a bottom portion of the middle section (340) of the recovery column (310);
maintaining a temperature of 105 to 125 °C in a bottom portion of a bottom section (350) of the recovery column (310); and
separating the acrylonitrile-water azeotrope from the acetonitrile to provide an overhead stream (303) that includes the acrylonitrile-water azeotrope, a bottoms stream (302), and a sidestream (306), and wherein
the recovery column (310) includes a crude acetonitrile concentration zone (342) which includes an internal vertical baffle (344) and an upper outlet configured to allow the sidestream (306) to flow out of the recovery column (310) from the crude acetonitrile concentration zone (342), and
further wherein
the overhead stream (303) includes the acrylonitrile-water azeotrope, 0.05 weight percent or less acetonitrile and has 70 to 90 weight percent acrylonitrile:
the bottoms stream (302) includes 0 to 0.0075 weight percent acetonitrile; and
the sidestream (306) includes 5 to 70 weight % acetonitrile.

2. The process of claim 1 wherein the recovery column is a single column.

3. The process of claim 1 wherein the recovery column includes a first column and a second column.

4. The process of claim 1 wherein the top section, middle section and bottom section are each 25 to 40% of a height (tangent to tangent) of the recovery column.

5. The process of claim 1 wherein the middle section includes a top portion, middle portion and bottom portion, and preferably wherein the top portion, middle portion and bottom portion are each 25 to 40% of a height (tangent to tangent) of the middle section of the recovery column.

6. The process of claim 1 wherein the mixture of acrylonitrile and acetonitrile are provided to the middle section of the recovery column.

7. The process of claim 1 wherein the top portion of the middle section of the recovery column is maintained at 70 to 80 °C.

8. The process of claim 1 wherein the bottom portion of the middle section of the recovery column is maintained at 105 to 115 °C.

9. The process of claim 1 wherein the aqueous solvent is provided to a top section of the recovery column.

10. The process of claim 1 wherein the top section of the recovery column includes a top portion and a bottom portion that are each 40 to 60% of a height (tangent to tangent) of the top section of the recovery column.

11. The process of claim 1 wherein the top portion of the top section of the recovery column is maintained at 60 to 75 °C.

12. The process of claim 1 wherein the top portion and bottom portion of the top section of the recovery column have a temperature difference of 0 to 20 °C, preferably of 5 to 15 °C.

13. The process of claim 1 wherein the bottom section of the recovery column includes a top portion and a bottom portion that are each 40 to 60% of a height (tangent to tangent) of the bottom section of the recovery column.

14. The process of claim 1 wherein the bottom portion of the bottom section of the recovery column is maintained at 110 to 120 °C.

15. The process of claim 1 wherein the top portion and bottom portion of the bottom section of the recovery column have a temperature difference of 0 to 15 °C, preferably of 7 to 13 °C.

16. The process of claim 1 wherein the overhead stream includes 0.03 weight percent or less acetonitrile, and preferably 0.01 weight percent or less acetonitrile.

17. The process of claim 1 wherein the overhead stream has 75 to 85 weight percent acrylonitrile.

18. The process of claim 1 wherein the bottoms stream includes 0.0025 to 0.007 weight percent acetonitrile, and preferably 0.0025 to 0.005 weight percent acetonitrile.

19. The process of claim 1 wherein the sidestream includes 5 to 50 weight % acetonitrile.

## Patentansprüche

1. Verfahren zum Abtrennen von Acrylnitril aus einem Gemisch, das Acrylnitril und Acetonitril beinhaltet, das Verfahren umfassend:
Bereitstellen des Gemischs aus Acrylnitril und Acetonitril für mindestens eine Rückgewinnungssäule (310) und Inkontaktbringen des Gemisches aus Acrylnitril und Acetonitril mit wässrigem Lösungsmittel, um ein Acrylnitril-Wasser-Azeotrop bereitzustellen;
Aufrechterhalten einer Temperatur von 55 bis 80 °C in einem oberen Abschnitt eines oberen Teils (330) der Rückgewinnungssäule (310);
Aufrechterhalten einer Temperatur von 65 bis 85 °C in einem oberen Abschnitt eines mittleren Teils (340) der Rückgewinnungssäule (310) und einer Temperatur von 100 bis 120 °C in einem unteren Abschnitt des mittleren Teils (340) der Rückgewinnungssäule (310);
Aufrechterhalten einer Temperatur von 105 bis 125 °C in einem unteren Abschnitt eines unteren Teils (350) der Rückgewinnungssäule (310); und
Abtrennen des Acrylnitril-Wasser-Azeotrops von dem Acetonitril, um einen Überkopfstrom (303), der das Acrylnitril-Wasser-Azeotrop beinhaltet, einen Bodenstrom (302) und einen Seitenstrom (306) bereitzustellen, und wobei
die Rückgewinnungssäule (310) eine Rohacetonitril-Konzentrationszone (342) beinhaltet, die eine interne vertikale Prallplatte (344) und einen oberen Auslass beinhaltet, der so konfiguriert ist, dass er es dem Seitenstrom (306) ermöglicht, von der Rohacetonitril-Konzentrationszone (342) aus der Rückgewinnungssäule (310) herauszufließen, und
wobei ferner
der Überkopfstrom (303) das Acrylnitril-Wasser-Azeotrop, 0,05 Gewichtsprozent oder weniger Acetonitril beinhaltet und 70 bis 90 Gewichtsprozent Acrylnitril aufweist:
der Bodenstrom (302) 0 bis 0,0075 Gewichtsprozent Acetonitril beinhaltet; und
der Seitenstrom (306) 5 bis 70 Gewichtsprozent Acetonitril beinhaltet.

2. Verfahren nach Anspruch 1, wobei die Rückgewinnungssäule eine einzelne Säule ist.

3. Verfahren nach Anspruch 1, wobei die Rückgewinnungssäule eine erste Säule und eine zweite Säule beinhaltet.

4. Verfahren nach Anspruch 1, wobei der obere Teil, der mittlere Teil und der untere Teil jeweils 25 bis 40 % einer Höhe (Tangente zu Tangente) der Rückgewinnungssäule ausmachen.

5. Verfahren nach Anspruch 1, wobei der mittlere Teil einen oberen Abschnitt, einen mittleren Abschnitt und einen unteren Abschnitt beinhaltet und wobei der obere Abschnitt, der mittlere Abschnitt und der untere Abschnitt vorzugsweise jeweils 25 bis 40 % einer Höhe (Tangente zu Tangente) des mittleren Teils der Rückgewinnungssäule ausmachen.

6. Verfahren nach Anspruch 1, wobei das Gemisch aus Acrylnitril und Acetonitril dem mittleren Teil der Rückgewinnungssäule bereitgestellt wird.

7. Verfahren nach Anspruch 1, wobei der obere Abschnitt des mittleren Teils der Rückgewinnungssäule bei 70 bis 80 °C aufrechterhalten wird.

8. Verfahren nach Anspruch 1, wobei der untere Abschnitt des mittleren Teils der Rückgewinnungssäule bei 105 bis 115 °C aufrechterhalten wird.

9. Verfahren nach Anspruch 1, wobei das wässrige Lösungsmittel einem oberen Teil der Rückgewinnungssäule bereitgestellt wird.

10. Verfahren nach Anspruch 1, wobei der obere Teil der Rückgewinnungssäule einen oberen Abschnitt und einen unteren Abschnitt beinhaltet, die jeweils 40 bis 60 % der Höhe (Tangente zu Tangente) des oberen Teils der Rückgewinnungssäule ausmachen.

11. Verfahren nach Anspruch 1, wobei der obere Abschnitt des oberen Teils der Rückgewinnungssäule bei 60 bis 75 °C aufrechterhalten wird.

12. Verfahren nach Anspruch 1, wobei der obere Abschnitt und der untere Abschnitt des oberen Teils der Rückgewinnungssäule eine Temperaturdifferenz von 0 bis 20 °C, vorzugsweise von 5 bis 15 °C, aufweisen.

13. Verfahren nach Anspruch 1, wobei der untere Teil der Rückgewinnungssäule einen oberen Abschnitt und einen unteren Abschnitt beinhaltet, die jeweils 40 bis 60 % der Höhe (Tangente zu Tangente) des unteren Teils der Rückgewinnungssäule ausmachen.

14. Verfahren nach Anspruch 1, wobei der untere Abschnitt des unteren Teils der Rückgewinnungssäule bei 110 bis 120 °C aufrechterhalten wird.

15. Verfahren nach Anspruch 1, wobei der obere Abschnitt und der untere Abschnitt des unteren Teils der Rückgewinnungssäule eine Temperaturdifferenz von 0 bis 15 °C, vorzugsweise von 7 bis 13 °C, aufweisen.

16. Verfahren nach Anspruch 1, wobei der Überkopfstrom 0,03 Gewichtsprozent oder weniger Acetonitril und vorzugsweise 0,01 Gewichtsprozent oder weniger Acetonitril beinhaltet.

17. Verfahren nach Anspruch 1, wobei der Überkopfstrom 75 bis 85 Gewichtsprozent Acrylnitril aufweist.

18. Verfahren nach Anspruch 1, wobei der Bodenstrom 0,0025 bis 0,007 Gewichtsprozent Acetonitril und vorzugsweise 0,0025 bis 0,005 Gewichtsprozent Acetonitril beinhaltet.

19. Verfahren nach Anspruch 1, wobei der Seitenstrom 5 bis 50 Gewichtsprozent Acetonitril beinhaltet.

## Revendications

1. Procédé de séparation de l'acrylonitrile d'un mélange qui comporte de l'acrylonitrile et de l'acétonitrile, le procédé comprenant :
la fourniture du mélange d'acrylonitrile et d'acétonitrile à au moins une colonne de récupération (310) et la mise en contact du mélange d'acrylonitrile et d'acétonitrile avec un solvant aqueux pour fournir un azéotrope acrylonitrile-eau ;
le maintien d'une température de 55 à 80 °C dans une partie supérieure d'une section supérieure (330) de la colonne de récupération (310) ;
le maintien d'une température de 65 à 85 °C dans une partie supérieure d'une section médiane (340) de la colonne de récupération (310) et d'une température de 100 à 120 °C dans une partie inférieure de la section médiane (340) de la colonne de récupération (310) ;
le maintien d'une température de 105 à 125 °C dans une partie inférieure d'une section inférieure (350) de la colonne de récupération (310) ; et
la séparation de l'azéotrope acrylonitrile-eau de l'acétonitrile pour fournir un courant de tête (303) qui comporte l'azéotrope acrylonitrile-eau, un courant de queue (302) et un courant latéral (306), et dans lequel
la colonne de récupération (310) comporte une zone de concentration d'acétonitrile brut (342) qui comporte un déflecteur vertical interne (344) et une sortie supérieure configurée pour permettre au courant latéral (306) de s'écouler hors de la colonne de récupération (310) à partir de la zone de concentration d'acétonitrile brut (342), et
également dans lequel
le courant de tête (303) comporte l'azéotrope acrylonitrile-eau, 0,05 pour cent en poids ou moins d'acétonitrile et a 70 à 90 pour cent en poids d'acrylonitrile :
le courant de queue (302) comporte 0 à 0,0075 pour cent en poids d'acétonitrile ; et
le courant latéral (306) comporte 5 à 70 % en poids d'acétonitrile.

2. Procédé selon la revendication 1, dans lequel la colonne de récupération est une colonne unique.

3. Procédé selon la revendication 1, dans lequel la colonne de récupération comporte une première colonne et une seconde colonne.

4. Procédé selon la revendication 1, dans lequel la section supérieure, la section médiane et la section inférieure représentent chacune 25 à 40 % d'une hauteur (tangente à tangente) de la colonne de récupération.

5. Procédé selon la revendication 1, dans lequel la section médiane comporte une partie supérieure, une partie médiane et une partie inférieure, et de préférence dans lequel la partie supérieure, la partie médiane et la partie inférieure représentent chacune 25 à 40 % d'une hauteur (tangente à tangente) de la section médiane de la colonne de récupération.

6. Procédé selon la revendication 1, dans lequel le mélange d'acrylonitrile et d'acétonitrile est fourni à la section médiane de la colonne de récupération.

7. Procédé selon la revendication 1, dans lequel la partie supérieure de la section médiane de la colonne de récupération est maintenue à 70 à 80 °C.

8. Procédé selon la revendication 1, dans lequel la partie inférieure de la section médiane de la colonne de récupération est maintenue à 105 à 115 °C.

9. Procédé selon la revendication 1, dans lequel le solvant aqueux est fourni à une section supérieure de la colonne de récupération.

10. Procédé selon la revendication 1, dans lequel la section supérieure de la colonne de récupération comporte une partie supérieure et une partie inférieure qui représentent chacune de 40 à 60 % d'une hauteur (tangente à tangente) de la section supérieure de ladite colonne de récupération.

11. Procédé selon la revendication 1, dans lequel la partie supérieure de la section supérieure de la colonne de récupération est maintenue à 60 à 75 °C.

12. Procédé selon la revendication 1, dans lequel la partie supérieure et la partie inférieure de la section supérieure de la colonne de récupération ont une différence de température de 0 à 20 °C, de préférence de 5 à 15 °C.

13. Procédé selon la revendication 1, dans lequel la section inférieure de la colonne de récupération comporte une partie supérieure et une partie inférieure qui représentent chacune de 40 à 60 % d'une hauteur (tangente à tangente) de la section inférieure de ladite colonne de récupération.

14. Procédé selon la revendication 1, dans lequel la partie inférieure de la section inférieure de la colonne de récupération est maintenue à 110 à 120 °C.

15. Procédé selon la revendication 1, dans lequel la partie supérieure et la partie inférieure de la section inférieure de la colonne de récupération ont une différence de température de 0 à 15 °C, de préférence de 7 à 13 °C.

16. Procédé selon la revendication 1, dans lequel le courant de tête comporte 0,03 pour cent en poids ou moins d'acétonitrile, et de préférence 0,01 pour cent en poids ou moins d'acétonitrile.

17. Procédé selon la revendication 1, dans lequel le courant de tête a 75 à 85 pour cent en poids d'acrylonitrile.

18. Procédé selon la revendication 1, dans lequel le courant de queue comporte de 0,0025 à 0,007 % en poids d'acétonitrile, et de préférence de 0,0025 à 0,005 % en poids d'acétonitrile.

19. Procédé selon la revendication 1, dans lequel le courant latéral comporte 5 à 50 % en poids d'acétonitrile.
